(19) Europäisches Patentamt
European Patent Office
Office européen des brevets

(11) **EP 4 407 304 A1**

(12) **EUROPEAN PATENT APPLICATION**
published in accordance with Art. 153(4) EPC

(43) Date of publication:
**31.07.2024  Bulletin 2024/31**

(21) Application number: **22901033.5**

(22) Date of filing: **07.11.2022**

(51) International Patent Classification (IPC):
*G01N 25/02* (2006.01)   *C10B 45/00* (2006.01)
*C10B 57/04* (2006.01)   *G01N 5/04* (2006.01)
*G01N 33/22* (2006.01)

(52) Cooperative Patent Classification (CPC):
**C10B 45/00; C10B 57/04; G01N 5/04; G01N 25/02;
G01N 33/22**

(86) International application number:
**PCT/JP2022/041426**

(87) International publication number:
**WO 2023/100600 (08.06.2023 Gazette 2023/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  03.12.2021   JP 2021196962

(71) Applicant: JFE Steel Corporation
Tokyo 100-0011 (JP)

(72) Inventors:
• **TANDOKORO, Kohei**
**Tokyo 100-0011 (JP)**
• **ISHIDA, Tomoharu**
**Tokyo 100-0011 (JP)**
• **INOSE, Masao**
**Tokyo 100-0011 (JP)**
• **ARAKAWA, Sara**
**Tokyo 100-0011 (JP)**
• **YAMAMOTO, Tetsuya**
**Tokyo 100-0011 (JP)**

(74) Representative: Hoffmann Eitle
**Patent- und Rechtsanwälte PartmbB
Arabellastraße 30
81925 München (DE)**

(54) **METHOD FOR CREATING COKE STRENGTH AFTER REACTION ESTIMATION MODEL,
METHOD FOR ESTIMATING COKE STRENGTH AFTER REACTION, AND COKE PRODUCTION
METHOD**

(57)    Provided is a method for establishing an estimation model for coke strength after reaction with which it is possible to rapidly estimate coke strength after reaction.

A method for establishing an estimation model for coke strength after reaction includes a step of obtaining coke strength after reaction, a step of obtaining a TG curve of the coke, and a model establishing step of establishing an estimation model for coke strength after reaction by using the TG curve obtained in the step of obtaining the TG curve of the coke as an explanatory variable and by using the strength after reaction obtained in the step of obtaining the coke strength after reaction as an objective variable.

FIG. 1

**Description**

Technical Field

The present invention relates to a method for establishing an estimation model for coke strength after reaction, a method for estimating coke strength after reaction, and a method for manufacturing coke.

Background Art

When pig iron is manufactured by using a blast furnace, coke is an indispensable material as a reducing agent for iron ore. The reason for this is because coke, which is porous, has gas permeability such that a gas blown from the lower part of the blast furnace passes through the coke to the upper part of the blast furnace.

To ensure sufficient gas permeability in a blast furnace, coke is required to have strength sufficient to suppress fracturing from occurring in the coke. Usually, coke strength is often represented by a value such as rotating drum strength or the like which is obtained by performing a test in air at room temperature. However, since the interior of a blast furnace is in a high-temperature environment whose temperature is higher than 1000°C and which contains a gas such as carbon dioxide that is reactive with coke, it may be said that the coke strength index obtained on the basis of the results of the ordinary test does not correctly indicate the coke strength in a blast furnace.

Here, as an index for accurately estimating the coke strength in a blast furnace, coke strength after reaction (CSR) is proposed, and a blast furnace is operated on the basis of the CSR. The expression "after reaction" denotes a situation where the coke has reacted with gases such as carbon dioxide and the like in a high-temperature environment at a temperature of higher than 1000°C after having been charged into a blast furnace. In addition, the term "coke strength after reaction" denotes the strength of coke after reaction.

For example, in the case where coke having a CSR which is lower than a target value is charged into a blast furnace, since the coke disintegrates into powder in the blast furnace, the flow of a reducing gas in the blast furnace is obstructed, and produced molten pig iron is inhibited from smoothly flowing down to the lower part of the blast furnace. Consequently, it is not possible to efficiently produce pig iron, and in some cases, a great deal of problems such as non-operation may occur. Therefore, it is important to correctly assess the CSR.

Measurement procedures for CSR are prescribed in the ISO standard "ISO 18894 Coal and Coke Test" and the ASTM standard "ASTM D 5341 Coal and Coke Test" regarding product specifications and testing methods (hereinafter, simply referred to as "ASTM standard"). For example, in the case of the method prescribed in the ASTM standard, after coke having a constant particle size has been held in a carbon dioxide atmosphere at a temperature of 1100°C for two hours, the coke is subjected to a rotating test utilizing an I-type drum tester, and the CSR is defined as a lump residual ratio after 600 revolutions. Usually, a lower limit of the CSR is set, and operation is performed while the CSR is controlled not to be lower than the lower limit.

An issue regarding the CSR measurement is the time required for obtaining the results of the CSR measurement. Although the time of the reaction between coke and carbon dioxide is two hours in the method prescribed in the ASTM standard as described above, since a furnace used for this reaction is large, a further long time is required for heating and cooling the furnace. In addition, the total time required for a series of operations including the coke preparation, the measurement performed using the drum tester after heating in the furnace, and the like may be almost one day.

In addition, even in the case where coke is subjected to the CSR analysis immediately after having been discharged from a coke oven in ordinary operation, the coke discharged from the coke oven has already been charged into a blast furnace before the results of CSR measurement are obtained. Therefore, since it is not possible to give an immediate response in blast furnace operation even in the case where an abnormal state of the coke indicated by the measurement results of the CSR is recognized, there are potential risks of a delay in a corrective action and unstable blast furnace operation. Therefore, there is a demand for a method for rapidly evaluating the CSR.

Regarding a method for rapidly evaluating the CSR, various investigations have been conducted. Patent Literature 1 discloses a method in which, by using Raman spectroscopy, the CSR is estimated by utilizing the intensity ratio of a certain peak.

In addition, investigations have also been conducted regarding the use of a coke reaction index (CRI), which has a high correlation with CSR. Patent Literature 2 discloses a method in which the CRI of coke made from a coal blend is determined on the basis of the CRI and total dilatation of relevant kinds of coke made from respective single coal brands by using a weighted average method and in which the CSR of the coke made from the coal blend is estimated on the basis of the determined CRI and drum strength of the coke made from the coal blend.

Moreover, Patent Literature 3 discloses a method in which the CSR is estimated by using the abundance ratios of inorganic constituents in coal and the physical property values of various raw material coals.

Citation List

Patent Literature

PTL 1: Japanese Unexamined Patent Application Publication No. 2019-163986
PTL 2: Japanese Unexamined Patent Application Publication No. 2005-232350
PTL 3: Japanese Unexamined Patent Application Publication No. 2001-172643

Summary of Invention

Technical Problem

However, in any of the estimation methods according to Patent Literature 1 to Patent Literature 3, it is not necessarily possible to obtain the analysis values and physical property values to be used in a short time, and a long time is required for obtaining some of the parameters to be used for estimation.
In addition, the estimation methods according to Patent Literature 2 and Patent Literature 3 utilize analysis values and physical property values which are obtained from coal before coking. However, in the case of these estimation methods, there is a problem in that operational errors which inevitably occur in the subsequent coking processes and variations in operational data due to such errors cause errors in the estimation of the CSR.
Moreover, the methods according to Patent Literature 2 and Patent Literature 3 in which a weighted average method or a multiple regression analysis method is used are intended for determining the blending ratio of single coal brands under the assumption that coke of a coal blend is made from single coal brands, and it is not possible to avoid the problem of the estimation error of the CSR. In addition, it is not possible to estimate the CSR of coke made from a coal blend in which the blending ratio of single coal brands is not known.
The present invention has been completed in view of the situation described above, and an object of the present invention is to provide a method for establishing an estimation model for coke strength after reaction with which it is possible to rapidly estimate coke strength after reaction.

Solution to Problem

The present inventors diligently conducted investigations and, as a result, completed the present invention by focusing on a weight decrease behavior on a TG curve (thermogravimetric change curve) when ground coke is analyzed by using a thermogravimetry (TG) method in a $CO_2$ atmosphere. The present invention has the following configurations.

[1] A method for establishing an estimation model for coke strength after reaction, the method including:

a step of obtaining coke strength after reaction;
a step of obtaining a TG curve of the coke; and
a model establishing step of establishing an estimation model for coke strength after reaction by using the TG curve obtained in the step of obtaining the TG curve of the coke as an explanatory variable and by using the strength after reaction obtained in the step of obtaining the coke strength after reaction as an objective variable.

[2] The method for establishing an estimation model for coke strength after reaction according to item [1], in which the step of obtaining the TG curve of the coke includes

a heating step of heating ground coke to a predetermined temperature range in an inert gas atmosphere and/or a $CO_2$ atmosphere, and
a holding step of, after the heating step, holding the coke in a $CO_2$ atmosphere at a holding temperature in the predetermined temperature range for a predetermined time.

[3] The method for establishing an estimation model for coke strength after reaction according to item [2], in which, in the heating step, the ground coke is heated to a temperature range of 900°C or higher and 1150°C or lower at a heating rate of 20°C/min or higher and 150°C/min or lower.
[4] The method for establishing an estimation model for coke strength after reaction according to any one of items [1] to [3], in which, in the model establishing step, the estimation model for coke strength after reaction is established by using partial least squares regression.

[5] A method for estimating coke strength after reaction, the method including estimating coke strength after reaction by using the estimation model for coke strength after reaction established by using the method for establishing an estimation model for coke strength after reaction according to any one of items [1] to [4].

[6] A method for manufacturing coke, the method including estimating coke strength after reaction by using the method for estimating coke strength after reaction according to item [5] and changing a condition for manufacturing coke in accordance with the estimated coke strength after reaction.

[7] The method for manufacturing coke according to item [6], in which changing the manufacturing condition is changing a coal blending ratio.

Advantageous Effects of Invention

According to the present invention, it is possible to provide a method for establishing an estimation model for coke strength after reaction with which it is possible to rapidly estimate coke strength after reaction.

According to the present invention, it is possible to increase not only rapidness in estimating coke strength after reaction, but also accuracy in estimating coke strength after reaction. As a result, it is possible to stabilize blast furnace operation. In addition, since it is possible to achieve an optimized coal blend in accordance with the results of estimating the coke strength after reaction, it is possible to improve the quality of coke and to stabilize coke oven operation.

Brief Description of Drawings

[Fig. 1] Fig. 1 is a TG curve of common coke which is obtained when, in a $CO_2$ atmosphere, the coke is heated from room temperature at a heating rate of 40°C/min and held at a temperature of 1120°C for two hours.

[Fig. 2] Fig. 2 is a graph illustrating the relationship between the CSR obtained by using a method in accordance with the ASTM standard and the CSR estimated by using the present invention.

Description of Embodiments

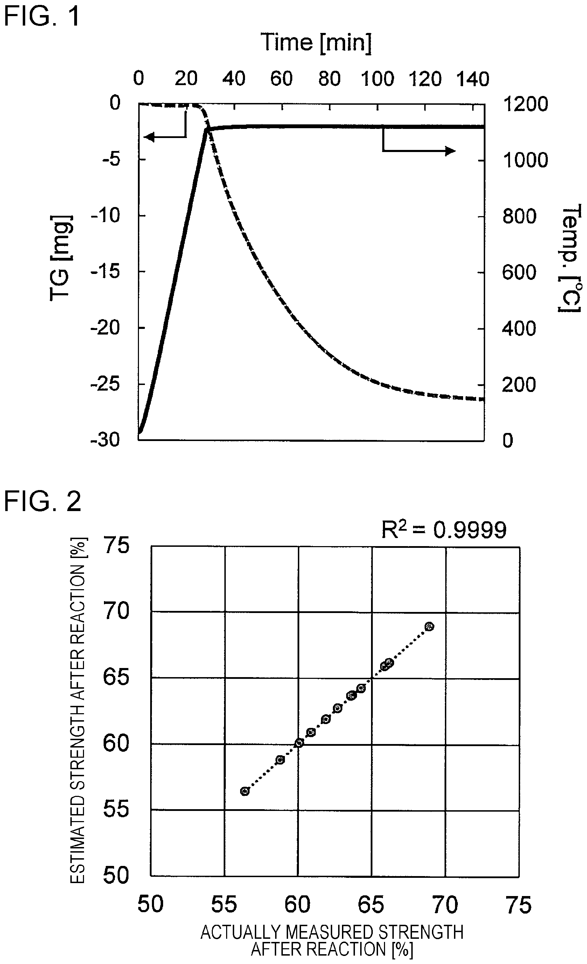

Fig. 1 is a TG curve of common coke which is obtained when, in a $CO_2$ atmosphere, the coke is heated from room temperature at a heating rate of 40°C/min and held at a temperature of 1120°C for two hours. First, in the process of heating to a temperature of 1120°C, a minute weight change is observed. This is because of a weight increase due to absorption of $CO_2$ by inorganic materials contained in the coke in an amount of about 10 mass% and a weight decrease due to thermal decomposition of the coke itself.

When the holding temperature is approached and holding is started at a predetermined temperature (1120°C in Fig. 1), a sharp weight decrease starts. This is mainly because of a gasification reaction in which carbon reacts with $CO_2$ to form CO. That is, this means that carbon which makes up the skeleton of coke is decomposed, and it is easy to assume that the strength also decreases accordingly. Therefore, it is considered that this TG curve (weight decrease behavior) is associated with the CSR.

It is said that the decomposition reaction of coke arises from a combination of various factors. For example, there are factors such as the structures of carbon constituents, the catalysis of inorganic materials, void structures having effects on the reaction surface area, and the like. Although the TG curve in Fig. 1 looks smooth, since it is considered that the TG curve is composed of such a complex combination of factors, the general shape of the TG curve varies in accordance with the brand, blending ratio, and production lot of coal, which is the raw material of coke.

The present inventors found that, by subjecting the TG curve, that is, the relationship between the time and weight from the early stage of the reaction to its closing stage, to machine learning, it is possible to estimate the CSR with high accuracy.

Hereafter, one embodiment for realizing the present invention will be described.

The method for establishing an estimation model for coke strength after reaction according to the present invention includes a step of obtaining coke strength after reaction, a step of obtaining a TG curve of the coke, and a model establishing step of establishing an estimation model for coke strength after reaction by using the TG curve obtained in the step of obtaining the TG curve of the coke as an explanatory variable and by using the strength after reaction obtained in the step of obtaining the coke strength after reaction as an objective variable.

[Step of obtaining coke strength after reaction]

In the step of obtaining coke strength after reaction, the coke strength after reaction (CSR) is actually measured. Specifically, the coke strength after reaction is actually measured in accordance with the ASTM standard. Although an example in which the coke strength after reaction is actually measured in accordance with the ASTM standard is described in the present embodiment, the coke strength after reaction may also be actually measured in accordance with, for example, the ISO standard described above or the like.

[Step of obtaining TG curve of coke]

In the step of obtaining the TG curve of the coke, the TG curve of the coke is obtained. That is, in this step, a TG curve of coke corresponding to (manufactured under the same conditions as) the coke used for actually measuring the coke strength after reaction in the step of obtaining coke strength after reaction is obtained. Either the step of obtaining the coke strength after reaction or the step of obtaining the TG curve of the coke may be performed first, or the two steps may be performed at the same time (concurrently).

In this step, as an apparatus for subjecting a coke sample to a thermal decomposition reaction, a thermogravimetry (TG) apparatus is used. Since the TG apparatus is excellent in terms of atmosphere control and temperature responsivity, it is possible to rapidly measure coke reaction index in a $CO_2$ atmosphere.

It is preferable that this step include a heating step of heating ground coke to a predetermined temperature range in an inert gas atmosphere and/or a $CO_2$ atmosphere and a holding step of, after the heating step, holding the coke in a $CO_2$ atmosphere at a holding temperature in the predetermined temperature range for a predetermined time.

It is preferable that the atmosphere in the holding step be a carbon dioxide ($CO_2$) atmosphere to simulate the interior of a blast furnace. This is the same environment as that used for the CSR analysis prescribed by ASTM and ISO. The heating step may be performed in an inert gas atmosphere and/or a $CO_2$ atmosphere. That is, the heating step may be performed in an inert gas atmosphere of $N_2$, Ar, He, or the like, a $CO_2$ atmosphere, or an atmosphere of a mixture thereof.

(Coke)

It is preferable that ground coke be used in this step. It is preferable that the coke be uniformly and finely powdered. In the case where the coke is not sufficiently ground, there is a possibility that it is not possible to correctly measure the weight decrease due to variations in the distributions of pores and inorganic materials in the coke. It is preferable that the particle size of the ground coke be 500 um or less, more preferably 200 um or less. The particle size of the ground coke may be adjusted by, for example, sieving the coke by using a sieve having a predetermined sieve opening.

The amount of coke used in this step may be adjusted in accordance with the capacity of the TG apparatus. To realize uniform heating, it is preferable that the amount of coke be about 50% of the capacity of a reaction container. Specifically, it is preferable that the amount of coke be 20 mg to 40 mg.

(Heating step)

In the TG apparatus, it is preferable that the coke be heated to a predetermined temperature range in an inert gas atmosphere of $N_2$, Ar, He, or the like and/or a $CO_2$ atmosphere (heating step). That is, in the heating step, it is preferable that the coke be heated to a predetermined temperature range in any of an inert gas atmosphere, a $CO_2$ ($CO_2$ gas) atmosphere, and an atmosphere of a mixture thereof. In the heating step and the holding step described below, it is preferable that the atmosphere gas be fed into the TG apparatus at a flow rate of 200 mL/min or higher. In the case where the flow rate is lower than this, since the amount of gas fed is insufficient in relation to the amount of coke, there is a possibility that it is not possible to correctly analyze a weight decrease due to a reaction.

(Holding step)

Subsequently, it is preferable that holding be performed at a holding temperature (soaking temperature) in a predetermined temperature range (holding step). It is preferable that the holding step be performed in a $CO_2$ atmosphere. In the case where a gas which is different from $CO_2$ is used in the heating step, it is preferable that the atmosphere gas be switched to $CO_2$ gas at the same time as the transition to the holding step. It is preferable that the predetermined temperature range be 900°C or higher and 1150°C or lower. It is more preferable that the predetermined temperature range be 950°C or higher. It is more preferable that the predetermined temperature range be 1125°C or lower. In the case where the temperature range is lower than 900°C, since the weight decrease rate is low, it is difficult to recognize the difference between samples. In addition, in the case where the temperature range is higher than 1150°C, since a decomposition reaction progresses sharply, there is a possibility that an analysis error occurs. The heating rate to the temperature range (heating rate from room temperature to the temperature range) may be appropriately adjusted in accordance with the capacity of the TG apparatus. It is preferable that the heating rate be 20°C/min or higher and 150°C/min or lower. In the case where the heating rate is lower than 20°C/min, since a long time is required for the temperature range to be reached, there is a decrease in the contribution to a method for rapid analysis. On the other hand, in the case where the heating rate is set to be higher than 150°C/min, since the decomposition reaction of inorganic materials, which occurs in a lower temperature range, and the decomposition reaction of the coke overlap each other due to the sharp temperature change, there is a possibility that it is difficult to correctly determine the TG curve attributed to the decomposition reaction of the coke. It is preferable that the heating rate be the heating rate from room temperature to the holding temperature in the predetermined temperature range. Here, room temperature is, for example, 25°C.

It is preferable that the predetermined time be 15 minutes or more and 75 minutes or less, which is sufficient for a sample (coke) charged into a TG apparatus to react. Specifically, it is preferable that the predetermined time be a period of time from when 15 minutes elapses until 75 minutes elapses after the temperature in the heating step has reached the predetermined temperature (holding temperature) and holding at the predetermined temperature has been started. In the case where the predetermined time is less than 15 minutes, since the thermal decomposition of inorganic materials and the like are included in the decomposition reaction, there is a possibility that an analysis error occurs. In addition, in the case where the predetermined time is more than 75 minutes, since the decomposition reaction of coke progresses sufficiently, it is difficult to recognize the difference in the TG curve, and there is a possibility that an analysis error occurs.

[Model establishing step of establishing estimation model for coke strength after reaction]

In the model establishing step of establishing an estimation model for coke strength after reaction, the estimation model for coke strength after reaction is established by using the TG curve obtained in the step of obtaining the TG curve of the coke as an explanatory variable and by using the strength after reaction obtained in the step of obtaining the coke strength after reaction as an objective variable.

Explanatory variables used for machine learning may include those correlated with each other to a certain extent. Due to collinearity caused by the correlative relationship, there may be a decrease in estimation accuracy in the case of simple multiple regression analysis. Also in the case of the analysis of coke, since it is not possible to deny the possibility that the explanatory variables are correlated with each other, it is desirable that an analysis method in which there is no problem of collinearity in principle be selected. One representative example of such an analysis method is partial least squares (PLS) regression.

In partial least squares regression, after the explanatory variables have been transformed into principle components which are not correlated with each other, correlation analysis is performed between the principle components and the objective variable; therefore, this method is advantageous for a case where strength after reaction is estimated from a plurality of analysis values whose explanatory variables are recognized to be correlated with each other, and provides high estimation accuracy.

In the present invention, as the explanatory variable, the TG curve obtained in the step of obtaining the TG curve of coke is used. Specifically, the explanatory variable is a TG curve which is obtained by heating ground coke in a $CO_2$ atmosphere in a predetermined temperature range for a predetermined time and by using a thermogravimetry method. That is, by using the strength after reaction obtained in the step of obtaining the coke strength after reaction as the objective variable and by using the TG curve obtained in the step of obtaining the TG curve of the coke as the explanatory variable, partial least squares regression analysis is performed. More specifically, by using the strength after reaction of the coke sample whose strength after reaction has been obtained as the objective variable and by using the TG curve of the corresponding coke sample as the explanatory variable, partial least squares regression analysis is performed to derive a model formula, which is a regression equation that defines the relationship between the objective variable and the explanatory variable. Subsequently, for a coke sample whose strength after reaction is unknown, by substituting a TG curve obtained from the sample (whose strength after reaction is unknown) into the derived model formula as the explanatory variable, strength after reaction, which is an objective variable, is calculated. That is, in the method for estimating coke strength after reaction according to the present invention, strength after reaction of coke whose strength after reaction is unknown is estimated by using the model formula (estimation model for coke strength after reaction) derived (established) as described above.

Here, as the explanatory variable, it is preferable to use the TG curve of the coke which is obtained while holding is performed at the holding temperature in the predetermined temperature range for the predetermined time in the step of obtaining the TG curve of the coke. As described above, it is preferable that the predetermined temperature range be 900°C or higher and 1150°C or lower. It is more preferable that the predetermined temperature range be 950°C or higher. In addition, it is more preferable that the predetermined temperature range be 1125°C or lower. It is preferable that the predetermined time be 15 minutes or more and 75 minutes or less, which is sufficient for a sample (coke) charged into a TG apparatus to react. Specifically, it is preferable that the predetermined time be a period of time from when 15 minutes elapses until 75 minutes elapses after the temperature in the heating step has reached the predetermined temperature (holding temperature) and holding at the predetermined temperature has been started.

Here, the actually measured (obtained) strengths after reaction of 13 kinds of coke sample (Nos. 1 to 13) which were used for partial least squares regression are given in Table 1. By establishing a model formula (equation 1) on the basis of partial least squares regression by using the TG curves of the coke samples given in Table 1 as the explanatory variables, an estimation model for coke strength after reaction was derived. Here, the TG curve of each of the coke samples which was used as the explanatory variable is data on time and weight measured at intervals of one second at 3601 time points in a period from when 15 minutes elapsed until 75 minutes elapsed after holding at the holding temperature (1120°C in this case) was started.

$$Y = c_0 + c_1 \times X_1 + c_2 \times X_2 + \cdots\cdots + c_n \times X_n$$

$$(\text{equation 1})$$

Here,

Y: CSR
$X_1$ to $X_n$: explanatory variable (measured TG data)
$c_0$ to $c_n$: regression coefficient obtained by expanding latent variable

The regression coefficients expressed by $c_0$ to $c_n$ define and weight the latent variables. The relationship between the latent variables and the regression equation is as follows.

$$Y = b_0 + b_1 \times T_1 + b_2 \times T_2 + \cdots\cdots + b_r \times T_r$$

Here,

Y: CSR
$T_1$ to $T_r$: latent variable
$b_0$ to $b_r$: coefficient of latent variable
where
first latent variable: $T_1 = w_{11} \times X_1 + w_{12} \times X_2 + \cdots + w_{1n} \times X_n$
second latent variable: $T_2 = w_{21} \times X_1 + w_{22} \times X_2 + \cdots + w_{2n} \times X_n$
r-th latent variable: $T_r = w_{r1} \times X_1 + w_{r2} \times X_2 + \cdots + w_{rn} \times X_n$
$w_{11}$ to $w_{rn}$: weighting of latent variable with respect to input variable
(Reference) Proceedings of the 54th Japan Joint Automatic Control Conference, 2K305 (2011)

[Table 1]

| No. | CSR |
|-----|------|
| 1 | 62.7 |
| 2 | 60.1 |
| 3 | 60.9 |
| 4 | 58.8 |
| 5 | 56.4 |
| 6 | 65.9 |
| 7 | 63.7 |
| 8 | 64.3 |
| 9 | 61.9 |
| 10 | 68.9 |
| 11 | 66.2 |
| 12 | 66.2 |

(continued)

| No. | CSR |
| --- | --- |
| 13 | 63.6 |

Fig. 2 illustrates the relationship between the coke strength after reaction which was estimated by substituting the TG curves of the coke samples given in Table 1 into the model formula and the coke strength after reaction which was obtained through actual measurement. In the figure, the horizontal axis represents the coke strength after reaction which was obtained through actual measurement (hereinafter, referred to as "actually measured strength after reaction"), the vertical axis represents the coke strength after reaction which was estimated (hereinafter, referred to as "estimated strength after reaction"). As illustrated in Fig. 2, the coefficient of determination ($R^2$) is 0.9999, which shows that there is a significantly high correlation between the two kinds of strength. Therefore, by using the model formula based on partial least squares regression, it is possible to estimate the coke strength after reaction with higher accuracy.

Here, the term "actually measured coke strength after reaction" denotes a value obtained by using the method in accordance with the ASTM standard as described above.

In a practical application, in the case where the coke strength after reaction which is estimated by using the estimation model for coke strength after reaction is out of the operationally acceptable range of the coke strength after reaction, it is possible to rapidly stop feeding the coke into a blast furnace, which results in stable blast furnace operation. In addition, since it is possible to efficiently perform blending ratio management such as changing the coal blending ratio for coke in accordance with the estimated coke strength after reaction, it is also possible to optimize the coal blending ratio. In addition, since it is also possible to rapidly change the conditions for manufacturing coke in accordance with the changed coal blending ratio, it is possible to improve the quality of coke and to stabilize coke oven operation. Here, changing the conditions for manufacturing coke in accordance with the estimated coke strength after reaction corresponds to the "method for manufacturing coke" according to the present invention. In addition, changing the manufacturing conditions for coke includes changing the coal blending ratio in accordance with the estimated coke strength after reaction.

As described above, in the present invention, an estimation model for coke strength after reaction is established in advance by using a method such as partial least squares regression, by using the TG curve obtained in the step of obtaining the TG curve of the coke as an explanatory variable, and by using the strength after reaction obtained in the step of obtaining the coke strength after reaction as an objective variable. In addition, by obtaining the TG curve of coke whose strength after reaction is unknown even when a blast furnace is in operation, it is possible to rapidly estimate the strength after reaction of the coke by only substituting the obtained TG curve into the estimation model for coke strength after reaction established in advance, and it is possible to rapidly determine whether or not the estimated strength after reaction is out of the operationally acceptable range of the coke strength after reaction. Moreover, by establishing the estimation model for the coke strength after reaction on the basis of partial least squares regression, it is possible to estimate the coke strength after reaction with higher accuracy. Here, the estimation of the coke strength after reaction utilizing the estimation model for the coke strength after reaction corresponds to the "method for estimating coke strength after reaction" according to the present invention.

In the method for estimating coke strength after reaction according to the present invention, since it is possible to estimate the CSR within about two to three hours, it is also possible to assess the CSR in real time during coke oven operation.

EXAMPLES

Hereafter, examples of estimating the coke strength after reaction by using the estimation model for coke strength after reaction according to the present embodiment will be described. However, the present invention is not limited to the examples described below.

Two kinds of coke (A and B) were prepared as samples. After 20 g of each kind of coke was weighed, the whole volume thereof was ground and sieved so as to have a particle size of 150 um or less. After air drying, 30 mg of the dried coke was weighed. As a TG apparatus, Thermo Plus 2 TG8120 produced by RIGAKU Corporation was used. The weighed coke was packed in a container made of platinum, which was attached to the apparatus, and charged into the apparatus. The atmosphere gas was $CO_2$, and the flow rate was 300 mL/min. After the apparatus had been stabilized, the coke was heated from room temperature to a temperature of 1120°C at a heating rate of 40°C/min, held at a temperature of 1120°C for 120 minutes, and then cooled to room temperature to measure the TG curve.

Then, the strength after reaction of each of the two kinds of coke (A and B) was estimated by substituting the TG curve of the coke into the estimation model for the coke strength after reaction, which had been established as a result of partial least squares regression performed on the basis of the strengths after reaction and TG curves of the 13 kinds of coke given in Table 1. In addition, the strength after reaction of each of the two kinds of coke (A and B) was actually measured in accordance with the ASTM standard, and the estimated strength after reaction and the actually measured strength after reaction were compared. The partial least squares regression was performed by using "OriginPro 2017" (registered trademark) and applying a cross-validation method. Here, the TG curve of each of the two kinds of coke (A and B) used for estimation of strength after reaction was taken in a period of 60 minutes from when 15 minutes elapsed until 75 minutes elapsed after holding at the holding temperature (1120°C) was started.

The results of the estimated strength after reaction and actually measured strength after reaction of each of the two kinds of coke (A and B) are given in Table 2. The actually measured strength after reaction and the estimated strength after reaction obtained by using partial least squares regression were in excellent agreement with each other.

[Table 2]

| No. | Actually measured strength after reaction | Estimated strength after reaction of Inventive Example | Actually measured value - estimated value |
|---|---|---|---|
| A | 62.1 | 63.5 | 1.49 |
| B | 67.7 | 67.3 | -0.41 |

As described above, according to the present invention, by establishing the estimation model for the coke strength after reaction in advance, it is possible to estimate the coke strength after reaction by only measuring the TG curve of the coke without measuring the coke strength after reaction in accordance with the ASTM standard each time. In addition, by using partial least squares regression as a method for multivariable analysis, it is possible to estimate the coke strength after reaction with higher accuracy.

In addition, while the time required for determining the coke strength after reaction (actually measured strength after reaction) by using the method in accordance with the ASTM standard was 9.5 hours in the Examples, the time required for estimating the coke strength after reaction by using the method according to the present invention was 3 hours. Therefore, the contribution of the method for estimating the coke strength after reaction according to the present invention is large in terms of time. In addition, it is expected that rapid evaluation of coke strength after reaction contributes to stabilizing operation and to stably manufacturing high-quality coke.

**Claims**

1. A method for establishing an estimation model for coke strength after reaction, the method comprising:

   a step of obtaining coke strength after reaction;
   a step of obtaining a TG curve of the coke; and
   a model establishing step of establishing an estimation model for coke strength after reaction by using the TG curve obtained in the step of obtaining the TG curve of the coke as an explanatory variable and by using the strength after reaction obtained in the step of obtaining the coke strength after reaction as an objective variable.

2. The method for establishing an estimation model for coke strength after reaction according to Claim 1, wherein the step of obtaining the TG curve of the coke comprises

   a heating step of heating ground coke to a predetermined temperature range in an inert gas atmosphere and/or a $CO_2$ atmosphere, and
   a holding step of, after the heating step, holding the coke in a $CO_2$ atmosphere at a holding temperature in the predetermined temperature range for a predetermined time.

3. The method for establishing an estimation model for coke strength after reaction according to Claim 2, wherein, in the heating step, the ground coke is heated to a temperature range of 900°C or higher and 1150°C or lower at a heating rate of 20°C/min or higher and 150°C/min or lower.

4. The method for establishing an estimation model for coke strength after reaction according to any one of Claims 1 to 3, wherein, in the model establishing step, the estimation model for coke strength after reaction is established by using partial least squares regression.

5. A method for estimating coke strength after reaction, the method comprising estimating coke strength after reaction by using the estimation model for coke strength after reaction established by using the method for establishing an estimation model for coke strength after reaction according to any one of Claims 1 to 4.

6. A method for manufacturing coke, the method comprising estimating coke strength after reaction by using the method for estimating coke strength after reaction according to Claim 5 and changing a condition for manufacturing coke in accordance with the estimated coke strength after reaction.

7. The method for manufacturing coke according to Claim 6, wherein changing the manufacturing condition comprises changing a coal blending ratio.

FIG. 1

FIG. 2

## INTERNATIONAL SEARCH REPORT

| International application No. |
| --- |
| **PCT/JP2022/041426** |

**A. CLASSIFICATION OF SUBJECT MATTER**

*G01N 25/02*(2006.01)i; *C10B 45/00*(2006.01)i; *C10B 57/04*(2006.01)i; *G01N 5/04*(2006.01)i; *G01N 33/22*(2006.01)i
FI: G01N25/02 Z; C10B57/04; C10B45/00 Z; G01N33/22 A; G01N5/04 A

According to International Patent Classification (IPC) or to both national classification and IPC

**B. FIELDS SEARCHED**

Minimum documentation searched (classification system followed by classification symbols)

G01N25/02; C10B45/00; C10B57/04; G01N5/04; G01N33/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | RODERO, J. I., Blast furnace and metallurgical coke's reactivity and its determination by thermal gravimetric analysis, Ironmaking & Steelmaking, Vol. 42, no. 8 , pp. 618-625<br>pp. 618-625 | 1, 4-7 |
| A | | 2-3 |
| A | JP 2018-177885 A (NIPPON STEEL & SUMITOMO METAL CORP.) 15 November 2018 (2018-11-15)<br>entire text, all drawings | 1-7 |
| A | JP 2020-200360 A (NIPPON STEEL CORP.) 17 December 2020 (2020-12-17)<br>entire text, all drawings | 1-7 |
| A | JP 11-116968 A (SUMITOMO METAL INDUSTRIES, LTD.) 27 April 1999 (1999-04-27)<br>entire text, all drawings | 1-7 |
| A | JP 2-302648 A (SUMITOMO METAL INDUSTRIES, LTD.) 14 December 1990 (1990-12-14)<br>entire text, all drawings | 1-7 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **05 January 2023** | **17 January 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
|---|
| **PCT/JP2022/041426** |

**C.** **DOCUMENTS CONSIDERED TO BE RELEVANT**

| Category* | Citation of document, with indication, where appropriate, of the relevant passages | Relevant to claim No. |
|---|---|---|
| A | JP 2019-70534 A (KRI INC.) 09 May 2019 (2019-05-09) <br>     entire text, all drawings | 1–7 |
| A | CN 104297282 A (WUHAN IRON & STEEL (GROUP) CORP.) 21 January 2015 (2015-01-21) <br>     entire text, all drawings | 1–7 |
| A | CN 103940697 A (UNIVERSITY OF SCIENCE AND TECHNOLOGY, BEIJING) 23 July 2014 (2014-07-23) <br>     entire text, all drawings | 1–7 |
| A | US 2016/0200579 A1 (GRAFTECH INTERNATIONAL HOLDINGS INC.) 14 July 2016 (2016-07-14) <br>     entire text, all drawings | 1–7 |

Form PCT/ISA/210 (second sheet) (January 2015)

**INTERNATIONAL SEARCH REPORT**
Information on patent family members

International application No.

**PCT/JP2022/041426**

| Patent document cited in search report | | | Publication date (day/month/year) | Patent family member(s) | | | Publication date (day/month/year) |
|---|---|---|---|---|---|---|---|
| JP | 2018-177885 | A | 15 November 2018 | (Family: none) | | | |
| JP | 2020-200360 | A | 17 December 2020 | (Family: none) | | | |
| JP | 11-116968 | A | 27 April 1999 | (Family: none) | | | |
| JP | 2-302648 | A | 14 December 1990 | (Family: none) | | | |
| JP | 2019-70534 | A | 09 May 2019 | (Family: none) | | | |
| CN | 104297282 | A | 21 January 2015 | (Family: none) | | | |
| CN | 103940697 | A | 23 July 2014 | (Family: none) | | | |
| US | 2016/0200579 | A1 | 14 July 2016 | EP entire text, all drawings CN | 3041810 105121386 | A1 A | |

Form PCT/ISA/210 (patent family annex) (January 2015)

**EP 4 407 304 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019163986 A
- JP 2005232350 A
- JP 2001172643 A

**Non-patent literature cited in the description**

- *Proceedings of the 54th Japan Joint Automatic Control Conference, 2K305,* 2011